# EUROPÄISCHE PATENTSCHRIFT

(11) **EP 1 926 700 B1**
(45) Veröffentlichungstag und Bekanntmachung des Hinweises auf die Patenterteilung: **01.03.2017**
(21) Anmeldenummer: 06776791.3
(22) Anmeldetag: 11.08.2006
(51) Int. Cl.: C08J 3/00, C07C 51/43

(54) **VERFAHREN ZUR HERSTELLUNG VON (METH)ACRYLSÄURE MIT SCHWERSIEDEAUFARBEITUNG DURCH KRISTALLISATION**
PROCESS FOR PRODUCING (METH)ACRYLIC ACID WITH TREATMENT OF THE HIGH-BOILING FRACTION BY CRYSTALLISATION
PROCEDE DE FABRICATION D'ACIDE METH(ACRYLIQUE) AVEC TRAITEMENT A POINT D'EBULLITION ELEVE PAR CRISTALLISATION

(30) Priorität: 17.08.2005 DE 102005039156
(43) Veröffentlichungstag der Anmeldung: 04.06.2008
(73) Patentinhaber: Evonik Degussa GmbH, 45128 Essen (DE)
(72) Erfinder: BALDUF, Torsten, 64319 Pfungstadt (DE); BUB, Günther, 45772 Marl (DE); MOSLER, Jürgen, 44577 Castrop-Rauxel (DE); SABBAGH, Andreas, 48249 Dülmen (DE); KOHN, Jürgen, 47495 Rheinberg (DE); SELBACH, Arndt, 67246 Dirmstein (DE)
(74) Vertreter: Lang, Arne
(86) Internationale Anmeldenummer: PCT/EP2006/007973
(87) Internationale Veröffentlichungsnummer: WO 2007/020024

(56) Entgegenhaltungen:
- EP-A1- 1 116 709
- WO-A-00/53560
- WO-A-2004/063134

## Beschreibung

Die Erfindung betrifft ein Verfahren zur Herstellung von (Meth)Acrylsäure.

"(Meth)Acrylsäure" wird in diesem Text für die Verbindungen mit den Nomenklaturnamen "Methacrylsäure" und "Acrylsäure" verwendet. Von beiden Verbindungen ist die Acrylsäure erfindungsgemäß bevorzugt.

(Meth)Acrylsäure kann durch verschiedene Verfahren hergestellt werden. Von besonderem Interesse sind Verfahren, die von petrochemischen Produkten wie Propylen ausgehen. In diesem Zusammenhang kann das Propylen zum einen durch katalytische Gasphasenoxidation oder auch Dampfphasenoxidation mit einem sauerstoffhaltigen Gas wie Luft zunächst zu Acrolein und im Anschluss zu Acrylsäure umgesetzt werden. Dabei wird in einem zweistufigen Prozess das Propylen zunächst auf katalytischem Wege zu Acrolein oxidiert, welches anschließend in einer zweiten Verfahrensstufe ebenfalls unter Einsatz von Katalysatoren zu Acrylsäure umgesetzt wird. Die so erhaltene Acrylsäure wird durch Absorption mit Wasser oder auch mit Hochsiedern zu einer wässrigen Lösung bzw. einer Hochsiederlösung aus dem gasförmigen Reaktionsgemisch entfernt. Anschließend erfolgt die Aufreinigung der Acrylsäure in der Regel auch azeotrope Destillation der Acrylsäurelösung, gefolgt von weiteren Reinigungsschritten. In vergleichbarer Weise kann die Synthese von Methacrylsäure durch katalytische Oxidation von Isobutylen, tert.-Butanol, Methacrolein oder Isobutylaldehyd in der Gasphase erfolgen.

Weiterhin gibt es Ansätze, die katalytische Reaktion nicht in der Gasphase sondern in Lösung durchzuführen. Hierbei kann das zum Einen mit homogenen und zum anderen mit heterogenen Katalysatoren geschehen, wie beispielsweise aus DE 102 01 783 A1 bekannt. Ein anderer Weg zur Herstellung von Acrylsäure geht zunächst von einem Polyol wie Glycerin aus, das zu Acrolein dehydratisiert wird, wie in DE 42 38 493 C1 beschrieben. Das so erhaltene Acrolein kann dann entweder durch Gasphasenoxidation oder durch Lösungsoxidation jeweils in Gegenwart von heterogenen oder homogenen Katalysatoren zu Acrylsäure umgesetzt werden.

EP 1 116 709 offenbart ein Verfahren zur Herstellung hochreiner Acrylsäure.

Den Produkten dieser verschiedenen Acrylsäure- bzw. Methacrylsäureherstellungsverfahren ist gemein, dass die Reinheit der in diesen Produkten vorliegenden (Meth)Acrylsäure in der Regel nicht ausreicht, um direkt zur Weiterverarbeitung in Polymere oder andere Folgeprodukte eingesetzt zu werden. Insbesondere im Zusammenhang mit der Herstellung von Superabsorbern, die im wesentlichen auf schwachvernetzter teilneutralsierter Polyacrylsäure basieren und von F.L. Buchholz und A.T. Graham in "Modern Superabsorbent Technology", Wiley-VCH, New York, 1998 näher beschrieben sind. Im Fall von der Herstellung von Superabsorbern, die meist in Hygieneartikel wie Windeln, Inkontinenzprodukte und Damenbinden oder Medizinprodukte wie Wundauflagen eingesetzt werden, werden an die Reinheit der (Meth)Acrylsäure besonders hohe Anforderungen gestellt. Dieses macht es notwendig, dass die Produkte der vorstehend beschriebenen (Meth)Acrylsäureherstellungsverfahren einem oder mehreren Aufreinigungsschritten unterzogen werden müssen. Bei der großtechnischen Herstellung von (Meth)Acrylsäure handelt es sich bei diesen Aufreinigungsschritten in aller Regel um eine oder mehrere Destillationen. Obgleich die Destillation als Aufreinigungsmethode großtechnisch gut beherrscht wird und sich seit langem bewährt hat, fallen bei der destillativen Aufreinigung durch das damit verbundene Erhitzen der (Meth)Acrylsäure häufig Dimere oder Oligomere an.

Um diese (Meth)Acrylsäure Dimere bzw. Oligomere in die entsprechenden Monomere zu überführen gibt es im Stand der Technik eine Reihe von Ansätzen.

So beschreibt beispielsweise JP Sho 61-36501 B2 eine thermische Spaltung der dimeren Acrylsäure bei gleichzeitiger Destillation der abgespaltenen, monomeren Acrylsäure. Dieses führt zu einer Abtrennung eines Großteils der hochsiedenden Komponenten, lässt jedoch die Konzentration an Maleinsäure, Maleinsäureanhydrid und Protoanemonin, die gleichzeitig als Verunreinigungen neben den (Meth)Acrylsäure Dimeren bzw. Oligomeren anfallen können, nahezu unberührt. Der so aufgearbeitete Strom wird dem Verfahren zur Herstellung von (Meth)Acrylsäure wieder zugeführt und bewirkt eine Anreicherung von Protoanemonin, Maleinsäure und Maleinsäureanhydrid in der fein gereinigten Acrylsäure, was deren Qualität vermindert und insbesondere sich auf eine Weiterverarbeitung dieser Acrylsäure in der radikalischen Polymerisation nachteilig auswirkt, da diese Verunreinigungen Kettenabbruchs- und Übertragungsreaktionen bei der radikalischen Polymerisation begünstigen.

Ferner beschreibt EP 0 887 334 A1 ein Verfahren zur Rückgewinnung von Acrylsäure aus einer Acrylsäure-Dimere, Acrylsäure und Maleinsäure beinhaltenden Zusammensetzung, wobei zunächst in einer Destillationsvorrichtung die Acrylsäure abgetrennt wird und das bei der Destillation erhaltene Sumpfprodukt, welches mit den Acrylsäure-Dimeren sowie mit der Acrylsäure angereichert ist, in einem dimeren Spaltreaktor überführt wird. Schließlich wird das im dimeren Spaltreaktor erhaltene Sumpfprodukt in die Destillationsvorrichtung zurückgeführt. Eine Reduzierung der Konzentration an Protoanemonin wird nicht erwähnt, obwohl Protoanemonin bei der weiteren Verarbeitung der Acrylsäure hinderlich ist. Außerdem führt die in der EP 0 887 334 A1 beschriebene Anordnung mit einem nachgeschalteten dimeren Spaltreaktor und mit teilweiser Rückführung des Kopfstromes des dimeren Spaltreaktors in die vorgeschaltete Destillationsvorrichtung zu einem unnötigen Anstieg des Zulaufstroms in die Destillationsvorrichtung und macht somit eine größere Dimensierung der Destillationsvorrichtung erforderlich.

Der vorliegenden Erfindung lag daher die Aufgabe zugrunde, die sich aus dem Stand der Technik ergebenden Nachteile zumindest teilweise zu lindern oder gar zu überwinden.

Weiterhin lag der vorliegenden Erfindung die Aufgabe zugrunde, die bei der Aufreinigung von Roh (Meth)Acrylsäure in dieser vorhandenen Verunreinigungen wie Dimere bzw. Oligomere und auch Maleinsäure, Maleinsäureanhydrid oder Protoanemonin in wirtschaftlicher Weise aufzuarbeiten.

Weiterhin lag eine erfindungsgemäße Aufgabe darin, eine Umwandlung der (Meth)Acrylsäure-Dimere bzw. Oligomere in (Meth)Acrylsäure mit verringerter Anreicherung von weiteren Verunreinigungen wie etwa Maleinsäure, Maleinsäureanhydrid oder Protoanemonin zu schaffen.

Ferner ist eine Vorrichtung zur Erzeugung hochreiner (Meth)Acrylsäure offenbart, die eine Aufreinigung von verunreinigter (Meth)Acrylsäure zu höchster Reinheit bei einem geringeren Energieaufwand, bei störungsfreieren und umweltschonenderen Betrieb und bei einem möglichst geringen Verlust an (Meth)Acrylsäure in Form von dimerer (Meth)Acrylsäure oder trimerer (Meth)Acrylsäure oder höheren Oligomeren zu erreichen.

Außerdem lag der Erfindung die Aufgabe zugrunde, ein Verfahren bereitzustellen, wobei bei der Herstellung und insbesondere bei der Aufreinigung von (Meth)Acrylsäure die Gefahr, dass es zu unkontrollierter Polymerisation von (Meth)Acrylsäure kommt, verringert wird.

Zudem lag eine erfindungsgemäße Aufgabe darin, durch geeignete Verfahrensverbesserungen die Herstellung von (Meth)Acrylsäure der Gestalt zu verbessern, dass die Herstellung von chemischen Produkten, die Polymere aus (Meth)Acrylsäure verwenden oder auf (Meth)Acrylsäure basieren, verbessert wird.

Mindestens einen Beitrag zur Lösung eines Teils dieser Aufgaben ergibt sich aus den kategoriebildenden Patentansprüchen, die nachfolgend im Einzelnen weiter erläutert werden. Weitere vorteilhafte Ausführungsformen und Weiterbildungen sind Gegenstand der jeweiligen abhängigen Ansprüche und der nachfolgenden Ausführung, die jeweils einzeln angewandt oder beliebig miteinander kombiniert werden können.

Das erfindungsgemäße Verfahren zur Herstellung von (Meth) Acrylsäure beinhaltet als Schritte
a) Synthese einer Roh-(Meth)Acrylsäurephase;
b) destillatives Aufarbeiten der Roh-(Meth)Acrylsäurephase unter Erhalt
   - einer (Meth)Acrylsäurephase, und
   - einer (Meth)Acrylsäure-Dimere oder (Meth)Acrylsäure-Oligomere oder beide beinhaltenden dimeren Phase;
c) Spalten mindestens eines Teils der (Meth)Acrylsäure-Dimere oder (Meth)Acrylsäure-Oligomere oder beiden aus der dimeren Phase unter Erhalt
   - einer (Meth)Acrylsäure beinhaltenden Leichtsiederphase, und
   - einer weniger als die Leichtsiederphase (Meth)Acrylsäure beinhaltenden Schwersiederphase;
d) Abtrennen mindestens eines Teils der (Meth)Acrylsäure aus der Leichtsiederphase über Kristallisation durch Bilden von einer oder mehreren Kristallen unter Erhalt
   - einer Rein-(Meth)Acrylsäure, und
   - eines Rückstands.

Die Roh-(Meth)Acrylsäurephase beinhaltet vorzugsweise in einem Bereich von 1 bis 80 Gew. %, vorzugsweise in einem Bereich von 5 bis 75 Gew. % und darüber hinaus bevorzugt in einem Bereich von 7 bis 70 Gew.-% (Meth)Acrylsäure, jeweils bezogen auf die Roh-(Meth)Acrylsäurephase. Die anderen Bestandteile der Roh-(Meth)Acrylsäurephase können zum einen Trägerstoffe wie Lösemittel, beispielsweise Wasser, oder höher als (Meth)Acrylsäure siedende Aromaten oder Trägergase oder mindestens zwei der vorstehenden Komponenten sein. Weiterhin weist die Roh-(Meth)Acrylsäurephase oftmals Verunreinigungen wie Dimere oder Oligomere der (Meth)Acrylsäure, Aldehyde, beispielsweise Acetaldehyd, Furfural oder Benzaldehyd, Propionsäure, Essigsäure, Maleinsäure, Maleinsäureanhydrid oder Protoanemonin auf. Dabei liegt der Gehalt an Aldehyden üblicherweise in einem Bereich von 100 bis 2.000 ppm, besonders bevorzugt in einem Bereich von 200 bis 1.000 ppm, der Gehalt an Maleinsäure und Maleinsäureanhydrid in einem Bereich von 500 bis 5.000 ppm, besonders bevorzugt in einem Bereich von 1.000 bis 2.000 ppm, der Gehalt an Dimeren oder Oligomeren in einem Bereich von 0,01 bis 2 Gew.-%, besonders bevorzugt in einem Bereich von 0,2 bis 0,6 Gew.-%, der Gehalt an Essigsäure in einem Bereich von 0,2 bis 10 Gew.-%, besonders bevorzugt in einem Bereich von 2 bis 4 Gew.-%, der Gehalt in Wasser in einem Bereich von 10 bis 50 Gew.-%, besonders bevorzugt in einem Bereich von 30 bis 40 Gew.-%, der Gehalt an Propionsäure bei weniger als 0,1 Gew.-%, besonders bevorzugt weniger als 0,05 Gew.-% und der Gehalt an Protoanemonin in einem Bereich von 10 bis 1.000 ppm, besonders bevorzugt in einem Bereich von 100 bis 200 ppm. Die Gewichtsmengen (in ppm oder in Gew.-%) sind jeweils auf das Gesamtgewicht der Roh-(Meth)Acrylsäurephase bezogen.

In dem erfindungsgemäßen Verfahren ist es bevorzugt, dass die Synthese gemäß Schritt a) ausgewählt ist aus der Gruppe bestehend aus i. Gasphasenoxidation, ii. Dehydratisierung eines mindestens eine OH-Gruppe aufweisenden organischen Moleküls, gegebenenfalls gefolgt von einer Oxidation, vorzugsweise Gasphasenoxidation, und iii. einer Flüssigphasenoxidation.

Der Syntheseweg der Gasphasenoxidation ist dem Fachmann allgemein bekannt und unter anderem in EP 1 319 648 A1 offenbart. Zur Dehydratisierung eignen sich neben Glycerin auch eine Hydroxycarbonsäure wie Milchsäure oder β-Hydroxypropionsäure als ein mindestens eine OH-Gruppe aufweisendes organisches Molekül.

Wenn Glycerin als ein solches Molekül eingesetzt wird, entsteht in der Dehydratisierungsreaktion Acrolein, das nachfolgend einer Oxidation, entweder einer Gasphasenoxidation oder einer Flüssigphasenoxidation, vorzugsweise einer Gasphasenoxidation unter Erhalt von Acrylsäure unterzogen werden kann. Im Fall der β-Hydroxypropionsäure wird durch die Dehydratisierung direkt Acrylsäure erhalten. Geeignete Dehydratisierungsbedingungen sind dem Fachmann bekannt und ergeben sich unter anderem aus DE 42 38 493 C1. Wege zur Flüssigphasenoxidation zur Herstellung von (Meth)Acrylsäure sind dem Fachmann gleichfalls bekannt und ergeben sich beispielsweise aus DE 102 01 783 A1.

In einer weiteren Ausgestaltung des erfindungsgemäßen Verfahrens ist es bevorzugt, dass die destillative Aufarbeitung gemäß Schritt b) in mindestens zwei aufeinander folgenden Destillationsschritten erfolgt. Hierzu wird die meist flüssige Roh-(Meth)Acrylsäurephase - in der Regel aus einer Absorption- oder Quenchkolonne stammend - in die erste Destillationskolonne zur Durchführung des ersten Destillationsschritts eingespeist und nach Durchführung des ersten Destillationsschritts die (Meth)Acrylsäure-reichere Phase einem weiteren Destillationsschritt unterzogen wird. Im Allgemeinen erfolgt die destillative Aufarbeitung in zwei, drei oder vier Destillationsschritten, wobei drei Destillationsschritte zwischen dem Absorptions- bzw. Quenchschritt und dem Spaltschritt c) bevorzugt sind. Die aus dem destillativen Aufarbeiten der Roh-(Meth)Acrylsäure erhaltene (Meth)Acrylsäurephase beinhaltet vorzugsweise mindestens 90 Gew.-%, besonders bevorzugt im Bereich von 92 bis 99,9 Gew.-% und darüber hinaus bevorzugt im Bereich von 95 bis 99,8 Gew.-% (Meth)Acrylsäure, jeweils bezogen auf die (Meth)Acrylsäurephase. Die ebenfalls bei der destillativen Aufarbeitung der Roh-(Meth)Acrylsäurephase anfallende dimere Phase basiert vorzugsweise auf
(α1) 0,1 bis 75 Gew.-%, besonders bevorzugt 5 bis 70 Gew.-% und darüber hinaus bevorzugt 10 bis 65 Gew.-% monomerer (Meth)Acrylsäure,
(α2) 1 bis 90 Gew.-%, besonders bevorzugt 10 bis 40 Gew.-% und darüber hinaus bevorzugt 20 bis 30 Gew.-% (Meth)Acrylsäure-Dimeren,
(α3) 1 bis 25 Gew.-%, besonders bevorzugt 2 bis 20 Gew.-% und darüber hinaus bevorzugt 5 bis 15 Gew.-% (Meth)Acrylsäure-Trimeren,
(α4) 0 bis 20 Gew.-%, besonders bevorzugt 1 bis 10 Gew.-% und darüber hinaus bevorzugt 2 bis 8 Gew.-% Wasser,
(α5) 1 bis 92 Gew.-%, besonders bevorzugt 10 bis 75 Gew.-% und darüber hinaus bevorzugt 40 bis 57 Gew.-% Oligomeren, die größer als (Meth)Acrylsäure-Trimere sind, sowie zu
(α6) 1 bis 20 Gew.-%, besonders bevorzugt 2 bis 15 Gew.-% und darüber hinaus bevorzugt 5 bis 10 Gew.-% weiteren, von den α1-, α2-, α3-, α4- und α5-Verbindungen verschiedenen Verbindungen, als Nebenprodukten, insbesondere Maleinsäureanhydrid,
wobei die Summe der Komponenten (α1) bis (α6) 100 Gew.-% beträgt.

Weiterhin ist es in dem erfindungsgemäßen Verfahren bevorzugt, dass das Spalten gemäß Schritt c) thermisch erfolgt. Dazu sind Temperaturen in einem Bereich von 50 bis 500°C, vorzugsweise in einem Bereich von 150 bis 400°C und besonders bevorzugt in einem Bereich von 250 bis 300°C besonders geeignet. Ferner hat es sich bewährt, die Spaltung bei einem Druck in einem Bereich von 0,1 bis 1000 bar, besonders bevorzugt bei einem Druck in einem Bereich von 10 bis 800 bar und darüber hinaus bevorzugt bei einem Druck in einem Bereich von 80 bis 600 bar durchzuführen. Somit ist es in dem erfindungsgemäßen Verfahren bevorzugt dass das Spalten gemäß Schritt c) unter einem von dem Umgebungsdruck verschiedenen Druck erfolgt. Der Umgebungsdruck ist hier der Druck, der aufgrund der jeweiligen Klimaverhältnisse an dem Ort der Spaltvorrichtung anzutreffen ist. Die Verweilzeit der dimeren Phase während des Spaltens in einem geeigneten Spaltungsbereich liegt vorzugsweise in einem Bereich von 0,1 bis 1 Stunde, bevorzugt in einem Bereich von 1 Sekunde bis 15 Minuten und darüber hinaus bevorzugt in einem Bereich von 1 Minute bis 10 Minuten.

Gemäß einer bevorzugten Ausführungsform des erfindungsgemäßen Verfahrens erfolgt das Spalten in Gegenwart eines Spaltmittels, vorzugsweise in Gegenwart von Wasser als Spaltmittel. Das Wasser und die (Meth)acrylsäure-Dimere oder (Meth)Acrylsäure-Oligomere werden dabei vorzugsweise in einem Gewichtsverhältnis Wasser : (Meth)acrylsäure-Dimere bzw. (Meth)Acrylsäure-Oligomere in einem Bereich von 0,01 : 1 bis 10 : 1, besonders bevorzugt in einem Bereich von 0,1 : 1 bis 8 : 1 und darüber hinaus bevorzugt in einem Bereich von 0,5 : 1 bis 6 : 1 eingesetzt.

In anderen besonderen Ausgestaltung des erfindungsgemäßen Verfahrens wird das Wasser in einer molaren Menge eingesetzt, die höchstens 90%, vorzugsweise höchstens 80% und darüber hinaus bevorzugt höchstens 50% der molaren Menge an (Meth)Acrylsäure beträgt, die in dimerer oder oligomerer Form gebunden ist (zwei (Meth)Acrylsäure-Moleküle in einem Dimer, drei (Meth)Acrylsäure-Moleküle in einem Trimer usw.).

In einer weiteren besonderen Ausgestaltung des erfindungsgemäßen Verfahrens wird das Wasser in einer molaren Menge eingesetzt, die mindestens 50%, vorzugsweise mindestens 80% und darüber hinaus bevorzugt mindestens 90% der molaren Menge der (Meth)Acrylsäure beträgt, die in dimerer oder oligomerer Form gebunden ist.

Im übrigen wird der Fachmann die zur Spaltung benötigte Menge des Wassers als Spaltmittel durch geeignete Vorversuche in einfacher Weise ermitteln. So wird der Fachmann solange Wasser zusetzen, bis bei den gewählten Druck- und Temperaturbedingungen eine möglichst vollständige Spaltung erfolgt ist bzw. bis auch bei einem weiteren Zusatz von Wasser keine Bildung monomerer (Meth)Acrylsäure mehr zu beobachten ist.

Im Falle der Herstellung und Aufarbeitung von Acrylsäure beinhaltet die bei der Spaltung anfallende Leichtsiederphase Acrylsäure-Dimere und Acrylsäure-Trimere vorzugsweise in einer Gesamtmenge in einem Bereich von 0 bis 10 Gew.-%, besonders bevorzugt in einer Gesamtmenge in einem Bereich von 0,1 bis 5 Gew.-% und darüber hinaus bevorzugt in einer Gesamtmenge in einem Bereich von 0,5 bis 1 Gew.-%, jeweils bezogen auf das Gewicht der Leichtsiederphase.

Die Schwersiederphase beinhaltet mindestens 5 Gew.-%, vorzugsweise mindestens 10 Gew.-% und darüber hinaus bevorzugt mindestens 20 Gew.-% weniger (Meth)Acrylsäure als die Leichtsiederphase.

Weiterhin ist in dem erfindungsgemäßen Verfahren bevorzugt, dass mindestens ein Teil der Leichtsiederphase in Schritt d) als Flüssigkeitsfilm über eine Kältequelle geführt wird. In diesem Zusammenhang ist es bevorzugt, dass die Kältequelle eine Oberflächentemperatur in einem Bereich von -40 bis 5°C, vorzugsweise in einem Bereich von -20 bis 0°C und darüber hinaus bevorzugt in einem Bereich von -10 bis -1°C aufweist. Das Führen des Flüssigkeitsfilms über die Kältequelle wird vorzugsweise so verstanden, dass die Kältequelle eine Oberfläche aufweist, mit der der Flüssigkeitsfilm beim Darüberführen in Kontakt kommt. Es ist weiterhin bevorzugt, dass der Flüssigkeitsfilm zum einen durch die Erdanziehungskraft über die Kältequelle geführt wird. Außerdem entspricht es einer Ausgestaltung der Erfindung, dass der Flüssigkeitsfilm durch Pumpkraft bewegt über die Kältequelle geführt wird. Ferner ist es möglich, dass der Flüssigkeitsfilm sowohl mittels Schwerkraft als auch durch Pumpleistung über eine Kältequelle geführt wird. Besonders bevorzugt ist es in dem erfindungsgemäßen Verfahren, dass der Flüssigkeitsfilm durch Erdanziehungskraft bewegt wird. Es in dem erfindungsgemäßen Verfahren besonders bevorzugt, dass mindestens ein Teil des Flüssigkeitsfilms als Fallfilm vorliegt.

Als Kältequelle kommen grundsätzlich alle dem Fachmann für die vorliegende Anwendung geeignet erscheinenden kälterzeugende Vorrichtungen in Betracht. So kann die Kältequelle gasförmig vorliegen, in dem ein gekühlter Gasstrom durch den Flüssigkeitsfilm geblasen wird oder der Flüssigkeitsfilm durch einen gekühlten Gasstrom geleitet wird. Es ist erfindungsgemäß jedoch bevorzugt, dass die Kältequelle mit einer festen Oberfläche ausgestaltet ist. Ferner ist es erfindungsgemäß bevorzugt, dass die Kältequelle mindestens teilweise flächig ausgebildet ist. Dadurch wird erreicht, dass der Flüssigkeitsfilm in Kontakt mit der mindestens teilweise ausgebildeten Fläche der Kältequelle zur Übertragung der Kälte der Kältequelle auf den Flüssigkeitsfilm in Kontakt kommen kann. Durch diese Maßnahme ist es ferner möglich, dass sich an der Fläche der Kältequelle ein oder mehrere Kristalle aus mindestens eines Bestandteils des Flüssigkeitsfilms ausbilden können. In einer bevorzugten erfindungsgemäßen Ausgestaltung wird so auf der Fläche der Kältequelle eine Kristallschicht ausgebildet, die wiederum durch den Flüssigkeitsfilm mindestens in Teilbereichen überströmt werden kann.

Ferner ist es in dem erfindungsgemäßen Verfahren bevorzugt, dass mindestens ein Teil der Kristalle einem Schwitzen unterzogen wird. Dieses Schwitzen wird im Allgemeinen dadurch erreicht, in dem die Temperatur der Kristalle langsam etwas erhöht wird. Vorzugsweise erfolgt diese Temperaturerhöhung leicht über den Kristallisations- oder Schmelzpunkt der Kristalle. Hierbei ist der Kristallisations- oder Schmelzpunkt der Kristalle um vorzugsweise mindestens 1°C, besonders bevorzugt 2 bis 20°C und darüber hinaus bevorzugt um 1,5 bis 5°C erhöht.

Im Allgemeinen wird bei dem Schwitzvorgang darauf geachtet, dass die zum Schwitzen gebrachten Kristalle nicht vollständig, sondern maximal zu einem kleinen Teil aufschmelzen. Dieser Teil beträgt vorzugsweise maximal 20 Gew.-%, besonders bevorzugt maximal 10 Gew.-% und darüber hinaus bevorzugt maximal 5 Gew.-% der Kristallmenge vor dem Beginn des Schwitzens.

Weiterhin ist es in dem erfindungsgemäßen Verfahren bevorzugt, dass die Kristalle nach dem Schwitzen geschmolzen werden. Das Schmelzen erfolgt im Vergleich zum Schwitzen bei einer höheren Temperatur als der Schwitztemperatur. Vorzugsweise liegt die Schmelztemperatur mindestens 1°C, vorzugsweise mindestens 5°C und darüber hinaus bevorzugt mindestens 10°C über der Schwitztemperatur. Die Temperaturmessungen können hier an der Grenzfläche zwischen Kältequelle und Kristallen vorgenommen werden. Im Zusammenhang mit der Aufreinigung von Acrylsäure ist es bevorzugt, dass die Schmelztemperatur im Bereich von 15 bis 50°C, besonders bevorzugt 30 bis 40°C liegt.

Weiterhin ist es in dem erfindungsgemäßen Verfahren bevorzugt, dass der Rückstand aus Schritt d) in einem Schritt e) durch eine weitere Kristallisation mindestens teilweise aufgetrennt wird in
- eine (Meth)Acrylsäure-reiche Phase, und
- eine Verunreinigungsphase.

Im Zusammenhang mit der weiteren Kristallisation ist es in dem erfindungsgemäßen Verfahren bevorzugt, dass die (Meth)Acrylsäure-reiche Phase mindestens teilweise in den Verfahrensschritt d) zugeführt wird. Durch diese Maßnahme kann eine deutliche Verbesserung des Einsatzfaktors erreicht werden.

Die ferner offenbarte Vorrichtung zur Herstellung von (Meth)Acrylsäure beinhaltet fluidleitend miteinander verbunden
- α: einen Synthesebereich,
- β: stromab des Synthesebereichs einen destillativen Aufarbeitungsbereich, beinhaltend eine im unteren Bereich des Aufarbeitungsbereichs ausgebildete Sumpfzone,
- γ: stromab der Sumpfzone einen beheizbaren Druckbehälter, beinhaltend eine obere Region und eine untere Region;
- δ: stromab der oberen Region einen Kristallisationsbereich beinhaltend einen ersten Auslass und mindestens einen weiteren Auslass.

Unter fluidleitend verbunden wird verstanden, dass die einzelnen Vorrichtungskomponenten und Einheiten so miteinander verbunden sind, dass Flüssigkeiten, Gase und Feststoffe zwischen den einzelnen Komponenten transportierbar sind. Vorzugsweise geschieht dieses durch Flüssigkeiten oder Gase führende Leitungssysteme.

Vorzugsweise ist der Synthesebereich nach Maßgabe der jeweils durchzuführenden Synthesewege in einer dem Fachmann für diese geläufigen Weise ausgestaltet. Im Fall der Gasphasenoxidation weist der Synthesebereich zwei miteinander verbundene Reaktoren auf, die einen Feststoffkatalysator aufweisen, der entweder als Pulver oder als Schicht oder als Kombination daraus vorzugsweise auf Platten oder in Rohren, die vorzugsweise bündelweise angeordnet sind, vorliegt. Im Fall der Dehydratisierung von Glycerin liegen gleichfalls zwei miteinander verbundene Reaktoren vor. Der erste ist als Dehydratisierungsreaktor in Form eines Druckbehälters ausgebildet. Der zweite Reaktor zur Umwandlung von Acrolein zu Acrylsäure ist ebenfalls, wie bereits bei der Gasphasenoxidation beschrieben, ausgestaltet. Bei der Dehydratisierung von β-Hydroxypropionsäure zu Acrylsäure wird nur ein Reaktor benötigt.

Der destillative Aufarbeitungsbereich ist vorzugsweise als eine oder mehrere Destillationskolonnen ausgestaltet, der sich insbesondere im Fall der Gasphasenoxidation an einen Quench- bzw. Absorptionsbereich anschließt.

Der Spaltbereich wird auch als Dimerenspalter bezeichnet und ist aus dem Fachmann bekannten druckbeständigen Materialien wie Edelstahl ausgeführt. Er ist insbesondere so ausgestaltet, dass er bei Drücken von mindestens 10 bar und Temperaturen von mindestens 200°C betrieben werden kann. Der Spaltbereich ist zudem vorzugsweise korrosionsbeständig.

Im Zusammenhang mit dem Kristallisationsbereich ist es bevorzugt, dass dieser eine mindestens teilweise flächig ausgestaltete Kühleinheit beinhaltet. Diese Kühleinheit kann aus einem, zwei oder mehreren Elementen bestehen. So ist es gemäß einer Ausgestaltung bevorzugt, die Kühleinheit aus Rohren zu bilden, wobei diese Rohre im Inneren von dem zu kühlenden Gut durchströmt werden und im äußeren Bereich von einem Kühl- bzw. Heizmittel umflossen werden. In einer anderen Ausgestaltung liegen die flächig ausgestalteten Kühleinheiten als Platten vor, die auch in Plattenserien angeordnet sein können. Diese Platten können wiederum Hohlräume aufweisen. Zum einen können diese Hohlräume von dem zu kühlenden Gut durchströmt werden. In diesem Fall würden die Platten durch ein Kühl- bzw. Heizmedium, das an den Außenflächen dieser Platten vorbeistreicht, auf die jeweils geeignete Temperatur gebracht. In einer anderen Form könnte das Kühlgut an den Außenflächen der Platten vorbeigeführt werden, wobei das Kühl- bzw. Heizmittel durch die Öffnungen in den Platten strömt.

Es ist ferner bevorzugt, dass der Kristallisationsbereich eine mindestens teilweise flächig ausgestaltete Heizeinheit beinhaltet. Zudem ist es erfindungsgemäß bevorzugt, dass der Kristallisationsbereich mindestens eine teilweise flächig ausgebildete Kühleinheit beinhaltet. Weiterhin ist es gemäß einer anderen Ausgestaltung bevorzugt, dass der Kristallisationsbereich eine mindestens teilweise flächig ausgestaltete Heiz/Kühleinheit beinhaltet. Durch das Vorsehen einer Heiz/Kühleinheit in dem Kristallisationsbereich ist es möglich, auf eine Kristallbildung an der Heiz/Kühleinheit einen Schwitz- und Schmelzvorgang folgen zu lassen. Daher ist es besonders bevorzugt, dass in dem Kristallisationsbereich die Heiz/Kühleinheit ortsgleich ausgebildet ist. Dieses kann beispielsweise durch eine Röhre erfolgen, die in ihrem Inneren das Kühlgut aufnimmt und von außen entweder durch ein Heizmittel oder durch ein Kühlmittel umströmt werden kann. So kann an ein und demselben Rohrabschnitt sowohl eine Kühlung zur Kristallbildung als auch ein Heizvorgang zum Schwitzen bzw. Schmelzen erfolgen. Besonders bevorzugte Kristallisationsbereiche mit den zu deren Betrieb hilfreichen Anlagenteilen sind bei der Firma Sulzer Chemtech AG, Schweiz, kommerziell erhältlich.

In einer anderen Ausgestaltung der Vorrichtung beinhaltet diese weiterhin ε stromab des mindestens einen weiteren Auslasses eine weitere Kristallisationseinheit. Als Kristallisationseinheit können alle dem Fachmann als geeignet erscheinende Kristallisationseinheiten eingesetzt werden, wie sie unter anderem ebenso von der Sulzer Chemtech AG oder auch von der Niro Process Technology BV, Niederlande, kommerziell erhältlich sind. Der Kristallisationsbereich wird vorzugsweise dynamisch, d.h. kontinuierlich, gefahren, während die Kristallisationseinheit statisch - auch als "batchweise" bezeichnet - betrieben wird.

Weiterhin offenbart wird ein Verfahren zur Herstellung eines Polymers beinhaltet die Schritte
I. Herstellen von Rein-(Meth)Acrylsäure nach einem erfindungsgemäßen Verfahren,
II. Polymerisieren einer diese Rein-(Meth)Acrylsäure beinhaltenden Monomerphase unter Erhalt einer Polymerphase,
III. Aufarbeiten der Polymerphase unter Erhalt eines Polymers.

Bei dem Polymer handelt es sich vorzugsweise um einen Superabsorber, in diesem Fall ist es empfehlenswert, dass das Acrylsäuremonomer mindestens teilweise mit einer Lauge, vorzugsweise Natronlauge, neutralisiert wird und dass bei der Polymerisation Vernetzer zugegen ist. Weitere Einzelheiten zur Herstellung eines Superabsorbers ergeben sich zusätzlich zu der oben genannten Referenz von Buchholz aus DE 40 20 780 C1. Ferner ist es bei dem Polymerisationsverfahren nicht zwingend notwendig, vollständig die Rein-(Meth)Acrylsäure aus dem erfindungsgemäßen Verfahren einzusetzen. Das Aufarbeiten der Polymerphase kann durch Fällung in einem das entstandene Polymer nicht lösenden Fällungsmittel, trocknen oder abdestillieren des in der Polymerisation verwendeten Lösungsmittels nach jeweils dem Fachmann geläufigen Methoden erfolgen.

Weiterhin betrifft die vorliegende Offenbarung Fasern, Folien, Formkörper, Lebensmittel- oder Futterzusatzstoffe, Arzneimittel, Kosmetika, Schäume, Superabsorber, Hygieneartikel, Papier-, Leder oder Textilhilfsmittel, beinhaltend oder basierend auf einer (Meth)Acrylsäure erhältlich nach einem erfindungsgemäßen Verfahren oder beinhaltend oder basierend auf einem Polymer erhältlich nach einem erfindungsgemäßen Polymerisationsverfahren.

Zudem betrifft die vorliegende Offenbarung die Verwendung einer (Meth)Acrylsäure erhältlich nach einem erfindungsgemäßen Verfahren oder eines Polymers erhältlich nach offenbarten Polymerisationsverfahren in oder für Fasern, Folien, Formkörper, Lebensmittel- oder Futterzusatzstoffe, Arzneimittel, Kosmetika, Schäume, Superabsorber, Hygieneartikel, Papier-, Leder- oder Textilhilfsmittel.

In diesem Zusammenhang ist die Nutzung der nach dem erfindungsgemäßen Verfahren hergestellten (Meth)Acrylsäure, insbesondere Acrylsäure, zur Herstellung von superabsorbierenden Polymeren hervorzuheben. Diese Superabsorber werden vorzugsweise in Hygieneartikeln wie Windeln, Inkontinenzprodukte und Damenhygieneartikel eingesetzt. Hierbei wird der Superabsorber vorzugsweise in Sauglagen, auch Cores genannt, nach dem Fachmann allgemein bekannten Verfahren eingearbeitet. Diese Cores werden wiederum zwischen eine flüssigkeitsdurchlässige Oberschicht und eine in der Regel flüssigkeitsundurchlässige Unterschicht des Hygieneartikels angeordnet.

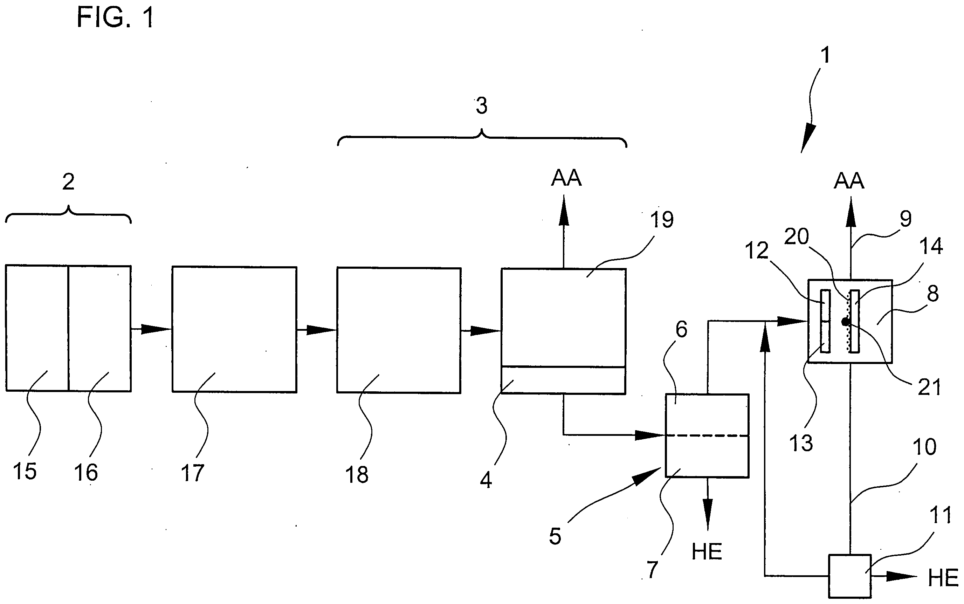

Weiterhin wird die vorliegende Erfindung durch nicht limitierende Zeichnungen beispielhaft erläutert. Hierbei zeigen
Fig. 1 eine schematische Darstellung den offenbarten Vorrichtung,

In Figur 1 weist eine Herstellvorrichtung 1 einen Synthesebereich 2 auf, der beispielsweise im Fall der Herstellung von Acrylsäure eine Acroleinreaktor 15 für die Gasphasenoxidation von Propen und einen Acrylsäurereaktor 16 für die Gasphasenoxidation von Acrolein aufweist. An den Acrylsäurereaktor 16 schließt sich eine Quencheinheit 17 an. Die darin gebildete Roh-Acrylsäure wird von dort in einen Aufarbeitungsbereich 3 mit einer ersten Kolonne 18 gefolgt von einer weiteren Kolonne 19 überführt. An einen Sumpfbereich 4 in der weiteren Kolonne 19 schließt sich ein als "Dimerenspalter" arbeitender Druckbehälter 5 an. Aufgereinigte Acrylsäure (AA) verlässt die weitere Kolonne 19 über Kopf. An einer oberen Region 6 des Druckbehälters 5 schließt sich ein Kristallisationsbereich 8 an. Bei der Spaltung anfallende Verunreinigungen (HE) werden aus einem unteren Bereich 7 des Druckbehälters 5 verworfen. Über einen ersten Auslass 9 wird die in dem Kristallisationsbereich 8 erhaltene aufgereinigten Acrylsäure (AA) ausgetragen. An einen weiteren Auslass 10 des Kristallisationsbereich 8 schließt sich eine Kristallisationseinheit 11 an, aus der Verunreinigungen (HE) verworfen und eine Acrylsäure reiche Phase dem Kristallisationsbereiches 8 wieder zugeführt wird. Der Kristallisationsbereich 8 weist eine Kühleinheit 12, eine Heizeinheit 13 und eine Heiz/Kühleinheit 14 auf, über die ein Fallfilm 20 geleitet wird, aus dem sich durch abkühlen Kristalle 21 bilden können, die durch aufheizen zunächst zum Schwitzen gebracht und dann geschmolzen werden können.

### Bezugszeichenliste

- 1: Herstellvorrichtung
- 2: Synthesebereich
- 3: Aufarbeitungsbereich
- 4: Sumpfzone
- 5: Druckbehälter
- 6: obere Region
- 7: untere Region
- 8: Kristallisationsbereich
- 9: erster Auslass
- 10: weiterer Auslass
- 11: Kristallisationseinheit
- 12: Kühleinheit
- 13: Heizeinheit
- 14: Heiz/Kühleinheit
- 15: Acroleinreaktor
- 16: Acrylsäurereaktor
- 17: Quencheinheit
- 18: erste Kolonne
- 19: weitere Kolonne
- 20: Fallfilm
- 21: Kristall

## Patentansprüche

1. Ein Verfahren zur Herstellung von (Meth)Acrylsäure, beinhaltend als Schritte
a) Synthese einer Roh(Meth)Acrylsäurephase;
b) destillatives Aufarbeiten der Roh(Meth)Acrylsäurephase unter Erhalt
- einer (Meth)Acryläurephase, und
- einer (Meth)Acrylsäure-Dimere oder (Meth)Acrylsäure-Oligomere oder beide beinhaltenden dimeren Phase;
c) Spalten mindestens eines Teils der (Meth)Acrylsäure-Dimere oder (Meth)Acrylsäure-Oligomere oder beiden aus der dimeren Phase unter Erhalt
- einer (Meth)Acrylsäure beinhaltenden Leichtsiederphase, und
- einer weniger als die Leichtsiederphase (Meth)Acrylsäure beinhaltenden Schwersiederphase;
d) Abtrennen mindestens eines Teils der (Meth)Acrylsäure aus der Leichtsiederphase über Kristallisation durch Bilden von ein oder mehreren Kristallen unter Erhalt
- einer Rein-(Meth)Acrylsäure, und
- eines Rückstands.

2. Verfahren nach Anspruch 1, wobei mindestens ein Teil der Leichtsiederphase in Schritt d) als Flüssigkeitsfilm über eine Kältequelle geführt wird.

3. Verfahren nach Anspruch 2, wobei mindestens ein Teil des Flüssigkeitsfilms als Fallfilm vorliegt.

4. Verfahren nach Anspruch 2 oder 3, wobei die Kältequelle mindestens teilweise flächig ausgebildet ist.

5. Verfahren nach einem der vorhergehenden Ansprüche, wobei mindestens ein Teil der Kristalle einem Schwitzen unterzogen werden.

6. Verfahren nach Anspruch 5, wobei die Kristalle nach dem Schwitzen geschmolzen werden.

7. Verfahren nach einem der vorhergehenden Ansprüche, wobei die Synthese gemäß Schritt a) ausgewählt ist aus der Gruppe, bestehend aus:
i. Gasphasenoxidation,
ii. Dehydratisierung eines mindestens eine OH-Gruppe aufweisenden organischen Molekels gegebenenfalls gefolgt von einer Oxidation,
iii. Flüssigphasenoxidation.

8. Verfahren nach einem der vorhergehenden Ansprüchen, wobei das destillative Aufarbeiten gemäß Schritt b) in mindestens zwei aufeinander folgenden Destillationsschritten erfolgt.

9. Verfahren nach einem der vorhergehenden Ansprüche, wobei das Spalten gemäß Schritt c) thermisch erfolgt.

10. Verfahren nach Anspruch 9, wobei das Spalten gemäß Schritt c) unter einem von dem Umgebungsdruck verschiedenen Druck erfolgt.

11. Verfahren nach einem der vorhergehenden Ansprüche, wobei der Rückstand aus Schritt d) in einem Schritt e) durch eine weitere Kristallisation mindestens teilweise aufgetrennt wird in
- eine (Meth)Acrylsäure-reiche Phase, und
- eine Verunreinigungsphase.

12. Verfahren nach Anspruch 11, wobei die (Meth)Acrylsäure-reiche Phase mindestens teilweise in den Verfahrensschritt d) zugeführt wird.

## Claims

1. Process for the production of (meth)acrylic acid, comprising the following steps:
a) synthesis of a crude (meth)acrylic acid phase;
b) distillative work-up of the crude (meth)acrylic acid phase to give
- a (meth)acrylic acid phase and
- a dimeric phase comprising (meth)acrylic acid dimers or (meth)acrylic acid oligomers or both;
c) separation of at least a portion of the (meth)acrylic acid dimers or (meth)acrylic acid oligomers or both from the dimeric phase to give
- a low-boiling-point phase comprising a (meth)acrylic acid and
- a high-boiling-point phase comprising less (meth)acrylic acid than the low-boiling-point phase;
d) isolation of at least a portion of the (meth)acrylic acid from the low-boiling-point phase by way of crystallization through formation of one or more crystals to give
- pure (meth)acrylic acid and
- a residue.

2. Process according to Claim 1, where at least a portion of the low-boiling-point phase in the form of liquid film in step d) is brought into contact with a means of heat abstraction.

3. Process according to Claim 2, where at least some of the liquid film takes the form of falling film.

4. Process according to Claim 2 or 3, where at least part of the means of heat abstraction has a defined, substantial surface area.

5. Process according to any of the preceding claims, where at least some of the crystals are subjected to a sweating process.

6. Process according to Claim 5, where the crystals are melted after the sweating process.

7. Process according to any of the preceding claims, where the synthesis according to step a) is selected from the group consisting of:
i. gas-phase oxidation,
ii. dehydration of an organic molecule having at least one OH group, optionally followed by oxidation,
iii. liquid-phase oxidation.

8. Process according to any of the preceding claims, where the distillative work-up according to step b) takes place in at least two successive distillation steps.

9. Process according to any of the preceding claims, where the separation according to step c) takes place thermally.

10. Process according to Claim 9, where the separation according to step c) takes place under a pressure differing from the ambient pressure.

11. Process according to any of the preceding claims, where, in a step e), further crystallization is used to divide at least part of the residue from step d) into
- a phase rich in (meth)acrylic acid and
- an impurity phase.

12. Process according to Claim 11, where at least part of the phase rich in (meth)acrylic acid is introduced into step d).

## Revendications

1. Procédé pour la préparation d'acide (méth)acrylique, contenant comme étapes
a) la synthèse d'une phase d'acide (méth)acrylique brut ;
b) le traitement par distillation de la phase d'acide (méth)acrylique brut avec obtention
- d'une phase d'acide (méth)acrylique et
- d'une phase dimère contenant des dimères d'acide (méth)acrylique ou des oligomères d'acide (méth)acrylique ou les deux ;
c) la dissociation d'au moins une partie des dimères d'acide (méth)acrylique ou des oligomères d'acide (méth)acrylique ou des deux de la phase dimère avec obtention
- d'une phase légère contenant de l'acide (méth)acrylique et
- une phase lourde contenant moins d'acide (méth)acrylique que la phase légère ;
d) la séparation d'au moins une partie de l'acide (méth)acrylique de la phase légère via une cristallisation par formation d'un ou de plusieurs cristaux avec obtention
- d'un acide (méth)acrylique pur et
- d'un résidu.

2. Procédé selon la revendication 1, au moins une partie de la phase légère dans l'étape d) étant guidée sous forme de film liquide sur une source de froid.

3. Procédé selon la revendication 2, au moins une partie du film liquide se trouvant sous forme de film tombant.

4. Procédé selon la revendication 2 ou 3, la source de froid étant conçue au moins en partie sous forme plane.

5. Procédé selon l'une quelconque des revendications précédentes, au moins une partie des cristaux étant soumise à un ressuage.

6. Procédé selon la revendication 5, les cristaux étant fondus après le ressuage.

7. Procédé selon l'une quelconque des revendications précédentes, la synthèse selon l'étape a) étant choisie dans le groupe constitué par :
i. l'oxydation en phase gazeuse,
ii. la déshydratation d'au moins une molécule organique présentant un groupe OH, le cas échéant suivie d'une oxydation,
iii. l'oxydation en phase liquide.

8. Procédé selon l'une quelconque des revendications précédentes, le traitement par distillation selon l'étape b) ayant lieu dans au moins deux étapes de distillation successives.

9. Procédé selon au moins l'une quelconque des revendications précédentes, la dissociation selon l'étape c) ayant lieu thermiquement.

10. Procédé selon la revendication 9, la dissociation selon l'étape c) ayant lieu sous une pression différente de la pression environnementale.

11. Procédé selon l'une quelconque des revendications précédentes, le résidu de l'étape d) étant séparé au moins partiellement dans une étape e) par une autre cristallisation en
- une phase riche en acide (méth)acrylique et
- une phase contenant des impuretés.

12. Procédé selon la revendication 11, la phase riche en acide (méth)acrylique étant alimentée au moins partiellement dans l'étape de procédé d).
